(19) Europäisches Patentamt
European Patent Office
Office européen des brevets

(11) Veröffentlichungsnummer: **0 284 914 B1**

(12) **EUROPÄISCHE PATENTSCHRIFT**

(45) Veröffentlichungstag der Patentschrift: **16.10.91**

(21) Anmeldenummer: **88104306.1**

(22) Anmeldetag: **18.03.88**

Die Akte enthält technische Angaben, die nach dem Eingang der Anmeldung eingereicht wurden und die nicht in dieser Patentschrift enthalten sind.

(51) Int. Cl.5: **C07D 405/06**, C07D 409/06, C07D 413/06, A61K 31/40, A61K 31/38, A61K 31/44, A61K 31/415, A61K 31/42, A61K 31/505

# (54) Benzofuranderivate und diese enthaltende therapeutische Mittel.

(30) Priorität: **30.03.87 DE 3710469**

(43) Veröffentlichungstag der Anmeldung: **05.10.88 Patentblatt 88/40**

(45) Bekanntmachung des Hinweises auf die Patenterteilung: **16.10.91 Patentblatt 91/42**

(84) Benannte Vertragsstaaten: **AT BE CH DE ES FR GB IT LI NL SE**

(56) Entgegenhaltungen:
**DE-A- 2 730 593**
**DE-B- 1 933 178**
**DE-B- 2 235 941**
**DE-B- 2 238 115**
**US-A- 3 631 034**

(73) Patentinhaber: **BASF Aktiengesellschaft**
**Carl-Bosch-Strasse 38**
**W-6700 Ludwigshafen(DE)**

(72) Erfinder: **Schlecker, Rainer, Dr.**
**Suedring 8**
**W-6719 Bissersheim(DE)**
Erfinder: **Ruebsamen, Klaus, Dr.**
**Erschigweg 19**
**W-6730 Neustadt(DE)**

**EP 0 284 914 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

CHEMICAL ABSTRACTS, Band 81, Nr. 17, 28. Oktober 1974, Seite 14, Zusammenfassung Nr. 99240k, Columbus, Ohio, US; G. RAYNAUD et al.: "Benzofurans. IV. Reduced chalcone derivatives with vasodilatory and antihypertensive action. Pharmacodynamic screening"

CHEMICAL ABSTRACTS, Band 81, Nr. 9, 2. September 1974, Seite 10, Zusammenfassung Nr. 45196g, Columbus, Ohio, US; G. RAYNAUD et al.: "Benzofurans. II. Chalcones with vasodilating effect. Pharmacological study"

A. BURGER "Medicinal Chemistry", John Wiley & Sons, Inc., New York, 1951, Seiten 76-77

**Beschreibung**

Die Erfindung betrifft neue Benzofuranderivate der allgemeinen Formel I und diese enthaltende therapeutische Mittel.

In den DE-B-22 35 941 und 22 38 115 sind für Benzofuranderivate der allgemeinen Formel I, in der anstelle von Het ein gegebenenfalls substituierter Phenylrest sitzt, unter elf verschiedenen pharmakologischen Wirkungen auch antiulceröse Eigenschaften erwähnt. Diese sind jedoch relativ schwach ausgeprägt. Folgerichtig sind in dem aus dem gleichen Labor stammenden späteren Bericht von G. Raynaud et al in Eur. J. Med. Chem. - Chimica Therapeutica, Edifor-Verl., Paris, 9 (1974), Seite 85 zwar siebenerlei Eigenschaften für diese Substanzklasse beschrieben, aber eine antiulceröse Wirkung nicht mehr erwähnt. Außerdem besitzen die Verbindungen eine unerwünschte Ca-antagonistische Aktivität.

Es wurde nun überraschend gefunden, daß Verbindungen der allgemeinen Formel I

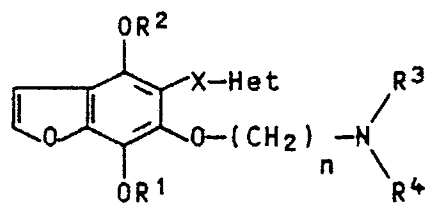

I,

in denen der Phenylring des Piprofurols durch einen Heterocyclus ersetzt ist, im Gegensatz zu den bekannten Verbindungen ähnlicher Struktur nur eine sehr schwache Ca-antagonistische Wirkung besitzen, aber stark antiulcerogen wirken.

In der allgemeinen Formel I stehen

| | |
|---|---|
| $R^1$ und $R^2$ | für Wasserstoff, Alkyl oder Phenylalkyl mit jeweils 1 bis 4 Kohlenstoffatomen im Alkylrest, |
| $R^3$ und $R^4$, | die gleich oder verschieden sind, für Wasserstoff oder $C_1$-$C_4$-Alkyl oder zusammen für eine 3- bis 5-gliedrige Kette, die durch ein Sauerstoffatom oder eine $NR^1$-Gruppe unterbrochen sein kann, |
| X | für $-CO-CH=CH-$, $-CO-CH_2-CH_2-$ oder $-CHOH-CH_2-CH_2-$, |
| n | für 2 oder 3 und |
| Het | für einen aromatischen 5- oder 6-gliedrigen Ring, der ein Heteroatom aus der Gruppe N, O und S und ggf. zusätzlich ein (weiteres) N-Atom enthält, jedoch nicht Pyridin, wobei der Ring subsituiert sein kann durch $C_1$-$C_4$-Alkyl, Fluor, Chlor, Brom, $NH_2$ oder $NR^1R^2$, wobei $R^1$ und $R^2$ die obengenannte Bedeutung haben. |

Als Heterocyclen seien beispielhaft genannt Pyrazol, Pyrrol, Thiophen, Furan, Isoxazol oder Pyrimidin.

Die Verbindungen der allgemeinen Formel I, in der X für die Gruppe $-CO-CH_2-CH_2-$ steht, können hergestellt werden aus den Verbindungen der Formel I, in denen X die Gruppe $-CO-CH=CH-$ bedeutet, durch katalytische Hydrierung der Doppelbindung nach literaturbekannten Methoden, wie sie beispielsweise in R.H. Rylander, "Catalytic Hydrogenation over Pt-Metals", Acad. Press, New York, S. 282, 1967, beschrieben sind. Als Katalysatoren eigenen sich insbesondere Metallkatalysatoren wie Palladium auf Kohle oder Raney-Nickel in Alkohol.

Die Verbindungen der allgemeinen Formel I, in denen X für die Gruppe $-CH(OH)-CH_2-CH_2-$ steht, können hergestellt werden durch Reduktion der Verbindungen der allgemeinen Formel I, in der X $-CO-CH_2-CH_2-$ bedeutet, mit Metallhydriden nach an sich bekannten Verfahren, wie sie in Houben-Weyl, Methoden der org. Chemie, 4. Auflage, G. Thieme Verlag Stuttgart 1984, Band 6/1 b, S. 145, beschrieben sind. Als Metallhydride eignen sich beispielsweise $LiAlH_4$ in Ethern oder $NaBH_4$ in Alkoholen wie Ethanol oder Isopropanol.

Weiterhin können diese Verbindungen hergestellt werden aus den Verbindungen der Formel I, in der X für $-CO-CH=CH-$ steht, durch katalytische Hydrierung, wie sie in Houben-Weyl Bd. 6/1b, S. 61 beschrieben ist, oder durch Reduktion mit Metallhydriden, wie sie in Houben-Weyl, 4. Aufl. (1981), Bd. 4/1d, S. 297 und in der DE-OS 22 35 941 beschrieben ist.

Die Synthese der Verbindungen der Formel I, in der X $CO-CH=CH_2$ bedeutet, erfolgt aus den Acetophenonen der Formel II durch Kondensation mit aromatischen heterocyclischen Aldehyden nach bekannten Methoden, wie sie beispielsweise in Org. Reactions Bd. 16, S. 1 ff, John Wiley Verlag, New York 1968 beschrieben sind. Als Kondensationsmittel können beispielsweise Alkalihydroxide in wäßrig alkoholischer Lösung dienen.

Die Verbindungen der Formel II, in der $R^1$ und $R^2$ = $CH_3$ ist, sind zum Teil bekannt und können erhalten werden durch Alkylierung des o-Hydroxyacetophenons Khellinon X (JACS 72 (1950), 1613) mit Halogenalkylaminen, wie es in Chimie Therapeutique 4 (1973), 475 beschrieben ist. Weiterhin kann die Alkylierung von X

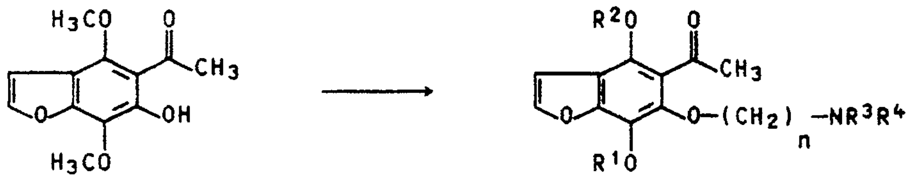

X II

mit Aminoalkoholen unter den Bedingungen der Mitsunobu-Methode (Synth. 1981, 1) erfolgen.

Die Verbindungen der Formel II, in denen $R^1$ oder $R^2 \neq CH_3$ ist, lassen sich herstellen durch Alkylierung der Hydroxyacetophenone III

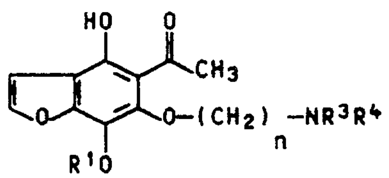

III

nach den an sich bekannten Methoden der Phenolsynthese (Houben-Weyl Bd. VI/3 S. 49 f.). So kann die Alkylierung beispielsweise durchgeführt werden mit Alkylhalogeniden unter Verwendung von Alkalicarbonaten als Base in Aceton als Lösungsmittel oder von Metallhydriden in aprotischen Lösungsmitteln, wie DMF oder THF.

Die Verbindungen III sind zugänglich durch Hydrogenolyse des Benzylethers IV nach bekannten Verfahren (Houben-Weyl Bd. 4/1c, S. 385 f.). Die Entbenzylierung wird vorzugsweise bei Raumtemperatur durchgeführt, um eine Hydrierung des Furanringes zu vermeiden.

Die Synthese der Verbindungen IV erfolgt aus den Hydroxyacetophenonen V nach den für die Herstellung von II ($R^1$ = $R^2$ = $CH_3$) beschriebenen Verfahren.

Das Hydroxyacetophenon V wird hergestellt durch alkalische Ringspaltung des Pyrons VI nach an sich bekannten Verfahren, wie sie für die Hydrolyse von Khellin in JACS 73, 1613 (1950) beschrieben sind.

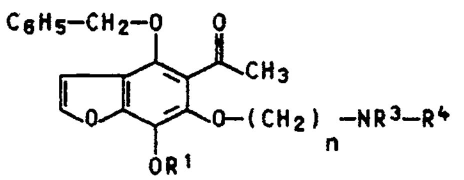

IV

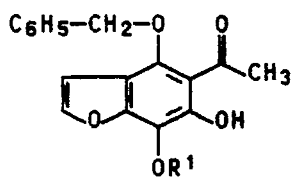

V

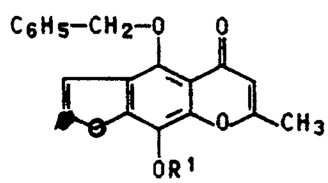

VI

Die Verbindung VI wird erhalten durch Benzylierung des Phenols VII nach allgemein bekannten Verfahren, wie sie für die Synthese von II ($R^1$ oder $R^2$ = $CH_3$) beschrieben sind.

Das Phenol VII mit $R^1$ = $CH_3$ kann synthetisiert werden nach einem literaturbekannten Verfahren (Liebigs Ann. 704, 182 (1967)) durch selektive Etherspaltung von Khellin mit KJ/Ameisensäure.

Das Phenol VII $R^1 \neq CH_3$ läßt sich herstellen durch selektive Alkylierung von Khellinchinol VIII, wie es in Ann. Pharm. Fr. 11, 685 (1953) beschrieben ist.

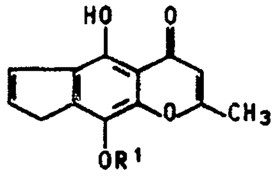

VII

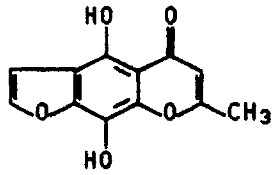

VIII

Die erfindungsgemäßen Verbindungen der Formel I, in denen X $CHOH-CH_2-CH_2$-bedeutet, besitzen ein Chiralitätszentrum und werden als Racemate erhalten, die durch bekannte Methoden, beispielsweise durch Bildung diastereomerer Salze mit optisch aktiven Hilfssäuren oder Dibenzoylweinsäure, Campher-10-Sulfonsäure oder Ditolylweinsäure in die optisch aktiven Antipoden getrennt werden können.

Gegebenenfalls werden die erhaltenen erfindungsgemäßen Verbindungen in das Säureadditionssalz einer physiologisch verträglichen Säure überführt. Eine Zusammenstellung üblicher physiologisch verträglicher Säuren kann aus Fortschritte der Arzneimittelforschung 1966, Birkhäuser Verlag, Bd. 10, S. 224 - 285, Deutschland, Schweiz, entnommen werden.

Die Säureadditionssalze werden in der Regel in an sich bekannter Weise durch Mischen der freien Base oder deren Lösungen mit der entsprechenden Säure oder deren Lösungen in einem organischen Lösungsmittel, beispielsweise einem niederen Alkohol, wie Methanol, Ethanol oder Propanol, oder einem niederen Keton, wie Aceton, Methylethylketon oder Methylisobutylketon, oder einem Ether, wie Diethylether, Tetrahydrofuran oder Dioxan, erhalten. Zur besseren Kristallabscheidung können auch Mischungen der genannten Lösungsmittel verwendet werden. Darüber hinaus können pharmazeutisch vertretbare wäßrige Lösungen von Säure-Additionsverbindungen der Aminopropanolderivate der Formel I durch Auflösen der freien Basen in einer wäßrigen Säurelösung hergestellt werden.

Die erfindungsgemäßen Verbindungen und ihre physiologisch verträglichen Säureadditionssalze sind wegen ihrer antiulcerosen und sekretionshemmenden Eigenschaften insbesondere zur Behandlung von Magenerkrankungen geeignet.

Gegenstand der vorliegenden Erfindung sind demnach auch therapeutische Mittel oder Zubereitungen, die neben pharmazeutisch üblichen Träger- und Verdünnungsmitteln eine Verbindung der Formel I oder eines ihrer physiologisch verträglichen Säureadditionssalze als Wirkstoff enthalten, sowie die Verwendung der neuen Verbindungen zu therapeutischen Zwecken.

Die therapeutischen Mittel bzw. Zubereitungen werden mit den üblichen Trägerstoffen oder Verdünnungsmitteln und den üblicherweise verwendeten pharmazeutisch-technischen Hilfsstoffen entsprechend der gewünschten Applikationsart mit einer geeigneten Dosierung in bekannter Weise hergestellt (vgl. R. Voigt, Lehrbuch der pharmazeutischen Technologie, VEB-Verlag Volk und Gesundheit, Berlin 1975). Als therapeutische Einzeldosen kommen Dosierungen von 1 bis 500 mg, bevorzugt 5 bis 100 mg, in Betracht.

Die bevorzugten Zubereitungen bestehen in einer Darreichungsform, die zur oralen Applikation geeignet ist. Solche Darreichungsformen sind Tabletten, Filmtabletten, Dragees, Kapseln, Pillen, Pulver, Lösungen oder Suspensionen oder Depotformen.

Selbstverständlich kommen auch parenterale Zubereitungen, wie Injektionslösungen, in Betracht. Weiterhin seien als Zubereitungen beispielsweise auch Suppositorien genannt. Zur praktischen Anwendung werden die erfindungsgemäß zu verwendenden Verbindungen mit den in der galenischen Pharmazie üblichen Trägerstoffen verarbeitet. Die entsprechenden Tabletten können beispielsweise unter Mitverwendung von inerten Verdünnungsmitteln, wie Dextrose, Zucker, Sorbit, Polyvinylpyrrolidon, Mannit, Calciumcarbonat, Calciumphosphat oder Milchzucker, Sprengmitteln, wie Maisstärke, Alginsäure oder Polyvinylpyrrolidon, Bindemitteln, wie Stärke oder Gelatine, Gleitmitteln wie Magnesiumstearat oder Talkum, und/oder Mitteln zur Erzielung des Depoteffektes, wie Celluloseacetphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummi arabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit den erfindungsgemäßen Wirkstoffen können zusätzlich geschmacksverbessernde Mittel, wie Saccharin, Cyclamat oder Zucker sowie z. B. Aromastoffe, wie Vanillin oder Orangeextrakt, enthalten. Sie können außerdem Suspendierhilfsmittel, wie Natriumcarboxymethylcellulose, oder Konservierungsstoffe, wie p-Hydroxybenzoate, enthalten. Wirkstoffe enthaltende Kapseln können

beispielsweise hergestellt werden, indem man den Wirkstoff mit einem inerten Träger, wie Milchzucker oder Sorbit, mischt und in Gelatinekapseln einkapselt.

Geeignete Suppositorien lassen sich beispielsweise durch Vermischen mit dafür vorgesehenen Trägermitteln, wie Neutralfetten oder Polyethylenglykol bzw. deren Derivaten und Formgebung herstellen.

A. Herstellung von Ausgangsverbindungen

4-Benzyloxy-9-methoxy-7-methyl-furo-[3,2-g]chromon (VI mit $R^2$ = $CH_3$)

95 g 4-Hydroxy-9-methoxy-7-methyl-furo[3,2-g]chromon werden in 1000 ml Methylethylketon mit 100 g Benzylbromid und 210 g $K_2CO_3$ 15 Stunden unter Rückfluß erhitzt. Die Mischung wird mit 1000 ml $CH_2Cl_2$ versetzt, filtriert und das Lösungsmittel abdestilliert. Der Rückstand liefert nach Behandeln mit Petrolether 123 g VI ($R^1$ = $CH_3$) als gelbliches Öl.

5-Acetyl-4-benzyloxy-6-hydroxy-7-methoxy-benzofuran (V mit $R^1$ = $CH_3$)

Zu einer Lösung von 67 g KOH in 1000 ml Wasser werden bei 75° C portionsweise 113g VI ($R^1$ = $CH_3$) gegeben. Die Mischung wird 3 Stunden unter Rückfluß erhitzt, abgekühlt und filtriert. Das Filtrat wird mit 110 ml konz. Salzsäure angesäuert, der ausgefallene Rückstand abgesaugt und getrocknet. Man erhält 115 g V ($R^1$ = $CH_3$).

5-Acetyl-4-benzyloxy-7-methoxy-6-(2-N-piperidinoethoxy)-benzofuran (allgemeine Formel IV)

50 g V ($R^1$ = $CH_3$) werden in 400 ml Methylethylketon mit 35 g Chlorethylpiperidin/90 g $K_2CO_3$ 5 Stunden unter Rückfluß erhitzt. Die Mischung wird filtriert, das Filtrat eingeengt. Der Rückstand wird zwischen Wasser und Ether verteilt. Man erhält 69 g IV ($R^1$ = $CH_3$) als gelbliches Öl.

5-Acetyl-4-hydroxy-7-methoxy-6-(2-N-piperidinoethoxy)benzofuran (allgemeine Formel III)

18,5 g IV ($R^1$ = $CH_3$ $R^3$ + $R^4$ gemeinsam = Pentamethylen) werden in 150 ml Essigester gelöst und in einer Hydrierapparatur mit 1 g Pd/C unter Normaldruck bei Raumtemperatur bis zur Beendigung der Wasserstoffaufnahme gerührt. Der Katalysator wird abfiltriert, das Filtrat eingeengt. Der Rückstand liefert 16 g des Zielproduktes.

5-Acetyl-4-ethoxy-7-methoxy-6-(2-piperidinoethoxy)benzofuran

5-Acetyl-4-hydroxy-7-methoxy-6-(2-piperidinoethoxy)-benzofuran in 2 ml DMF werden bei Raumtemperatur zu einer Suspension von 0,4 g NaH (55 % in Paraffinöl) in 10 ml DMF getropft. Nach 30 minütigem Rühren werden 2,1 g Ethyljodid zugegeben. Man läßt über Nacht rühren, gießt die Mischung auf Eis und extrahiert mit Ether. Die Etherphase wird mit Wasser gewaschen und eingeengt. Der Rückstand wird zwischen $CH_2Cl_2$ und 1 n HCl verteilt. Die org. Phase wird getrocknet und eingeengt. Man erhält 2,0 g des Hydrochlorids der Zielverbindung.

In analoger Weise wurden beispielsweise hergestellt:

5-Acetyl-4-propoxy-7-methoxy-6-(2-piperodinoethoxy)benzofuran; Öl
5-Acetyl-4,7-diethoxy-6-(2-piperidinoethoxy)benzofuran; Öl
5-Acetyl-4-ethoxy-7-methoxy-6-(3-piperidinopropoxy)benzofuran; Öl
5-Acetyl-4-ethoxy-7-methoxy-6-(2-morpholinoethoxy)benzofuran; Öl
5-Acetyl-4-ethoxy-7-methoxy-6-(2-dimethylaminoethoxy)-benzofuran; Öl
5-Acetyl-4-ethoxy-7-methoxy-6-(2-pyrrolidinoethoxy)benzofuran; Öl
5-Acetyl-4-ethoxy-7-methoxy-6-(2-(4-methylpiperazino)ethoxy)-benzofuran; Öl

Beispiele zur Herstellung der erfindungsgemäßen Verbindungen

Beipiel 1

1-[4,7-Dimethoxy-6-(2-piperidinoethoxy)benzofuranyl-5-]-3-(1-methyl-pyrazolyl-4)propenon

8,0 g 5-Acetyl-4,7-dimethoxy-6-(2-piperidinoethoxy)benzofuran und 2,6 g 1-Methylpyrazol-4-aldehyd

wurden in 60 ml Ethanol mit einer Lösung von 11 g NaOH in 11 ml Wasser versetzt. Die Mischung wurde über Nacht gerührt, auf Wasser gegossen und mit $CH_2Cl_2$ extrahiert. Nach Abziehen des Lösungsmittels erhielt man 9.0 g eines gelblichen Öls. Dieses wurde in Methyl-tert.-butylether gelöst und durch Zugabe etherischer Salzsäure als Hydrochlorid gefällt. Ausbeute 8,5 g Fp. 78 - 80 °C.

In analoger Weise wurden die Beispiele in Tabelle 1 synthetisiert. Die Strukturen aller Verbindungen wurden NMR-spektroskopisch gesichert.

Beispiel 19

1-(4-Ethoxy-7-methoxy-6-(2-piperidinoethoxy)benzofuranyl-5-)-3-(1-methylpyrazolyl-4-)propanon

6,6 g 1-(4-Ethoxy-7-methoxy-6-(2-piperidinoethoxy)benzofuranyl-5-)-3-(1-methylpyrazolyl-4-)propenon wurden in 200 ml Methanol mit 0,8 g Pd/C (10 %) unter Normaldruck bis zur Aufnahme der berechneten Wasserstoffmenge hydriert. Der Katalysator wurde abfiltriert, das Filtrat eingeengt und der Rückstand chromatographiert ($CH_2Cl_2$/Methanol 1 : 1). Ausbeute: 3,3 g (Öl).

Analog wurden hergestellt:

Beispiel 20

1-(4-Ethoxy-7-methoxy-6-(2-piperidinoethoxy)benzofuranyl-5-)-3-(1-ethylpyrazolyl-4-)propanon. Die Substanz wurde als Öl erhalten.

Beispiel 21

1-(4-Ethoxy-7-methoxy-6-(2-piperidinoethoxy)benzofuranyl-5-)-3-(thienyl3-)propanon.
Die Substanz wurde als Öl erhalten.

Beispiel 22

1-(4-Ethoxy-7-methoxy-6-(2-piperidinoethoxy)benzofuranyl-5-)-3-(thienyl-3) propanol

9 g 1-(4-Ethoxy-7-methoxy-6-(2-piperidinoethoxy)benzofuranyl-5-)-3-(thienyl-3-)propanon wurden in 30 ml Isopropanol mit 3,3 g $NaBH_4$ 6 Stunden unter Rückfluß erhitzt. Die Mischung wurde auf Eiswasser gegossen und mit $CH_2Cl_2$ extrahiert. Die org. Phase wurde getrocknet, das Lösungsmittel abdestilliert und der Rückstand nach Behandeln mit Petrolether in Ether gelöst. Zugabe der equivalenten Mengen etherischer HCl lieferte 1,3 g Produkt als Hydrochlorid. Fp. 138 - 139 °C.

Beispiel 23

1-(4-Ethoxy-7-methoxy-6-(2-piperidinoethoxy)benzofuranyl-5-)-3-(1-methylpyrrolyl-3)propanol

9,7 g Propenon aus Beispiel 6 wurden in 30 ml Isopropanol/7 ml Pyridin mit 3,4 g $NaBH_4$ und wenigen Tropfen conc. NaOH versetzt und 10 Stunden zum Rückfluß erhitzt. Die Mischung wurde einrotiert, der Rückstand zwischen $CH_2Cl_2$ und Wasser verteilt, die org. Phase abgetrennt, mit $Na_2SO_4$ getrocknet und das Lösungsmittel abgezogen. Nach Digerieren mit Petrolether wurden 2,5 g Produkt erhalten. Fp. 88 °C.

Analog wurden die Verbindungen von Tabelle 2 hergestellt.

Tabelle 1

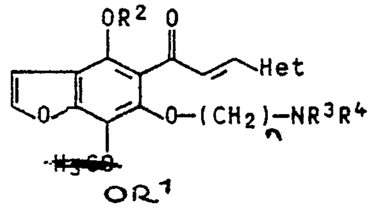

| Beisp. Nr. | $R^1$ | $R^2$ | n | $NR^3R^4$ | Het | Fp [°C] |
|---|---|---|---|---|---|---|
| 2 | $CH_3$ | $CH_3$ | 2 | 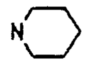 | 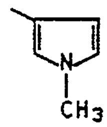 | 115-117 (Oxalat) |
| 3 | $CH_3$ | $CH_3$ | 2 | 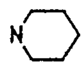 | 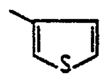 | 126-127 (Oxalat) |
| 4 | $CH_3$ | $CH_3$ | 2 | 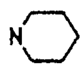 | 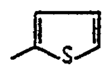 | 118-120 (Oxalat) |
| 5 | $CH_3$ | $C_2H_5$ | 2 | 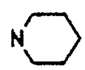 | 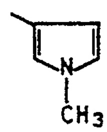 | Öl |
| 6 | $CH_3$ | $C_2H_5$ | 2 | 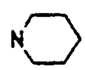 | 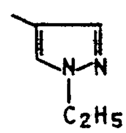 | 42 (Oxalat) |
| 7 | $CH_3$ | $C_2H_5$ | 2 | 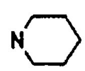 | 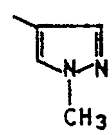 | Öl |
| 8 | $CH_3$ | $C_6H_5-CH_2$ | 2 | 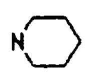 | 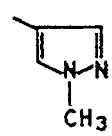 | Öl |

Tabelle 1 (Fortsetzung)

| Beisp. Nr. | $R^1$ | $R^2$ | n | $NR^3R^4$ | Het | Fp [°C] |
|---|---|---|---|---|---|---|
| 9 | $CH_3$ | $C_2H_5$ | 2 | 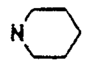 | 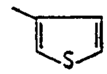 | Öl |
| 10 | $CH_3$ | $C_3H_7$ | 2 | 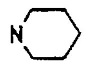 | 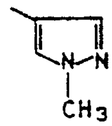 | Öl |
| 11 | $CH_3$ | $C_2H5$ | 2 | 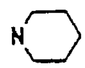 | 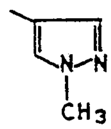 | Öl |
| 12 | $CH_3$ | $C_2H_5$ | 2 | 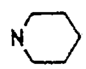 | 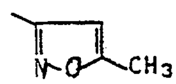 | Öl |
| 13 | $CH_3$ | $C_2H_5$ | 2 | 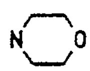 | 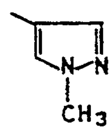 | Öl |
| 14 | $CH_3$ | $C_2H_5$ | 2 | 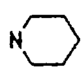 | 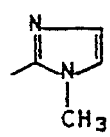 | Öl |
| 15 | $CH_3$ | $C_2H_5$ | 3 | 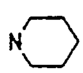 | 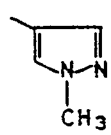 | Öl |

Tabelle 1 (Fortsetzung)

| Beisp. Nr. | $R^1$ | $R^2$ | n | $NR^3R^4$ | Het | Fp [°C] |
|---|---|---|---|---|---|---|
| 16 | $CH_3$ | $C_2H_5$ | 2 | 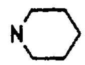 | 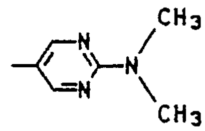 | 143-144 |
| 17 | $CH_3$ | $C_2H_5$ | 2 | 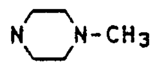 | 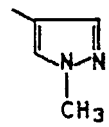 | Öl |
| 18 | $CH_3$ | $C_2H_5$ | 2 | 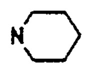 | 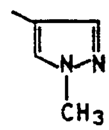 | Öl |

Tabelle 2

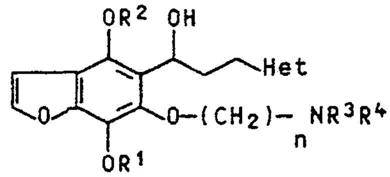

| Beisp. Nr. | $R^1$ | $R^2$ | n | $NR^3R^4$ | Het | Fp [°C] |
|---|---|---|---|---|---|---|
| 24 | $CH_3$ | $CH_3$ | 2 | 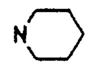 | 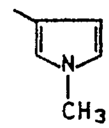 | 57- 58 (Oxalat) |
| 25 | $CH_3$ | $CH_3$ | 2 | 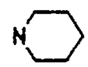 | 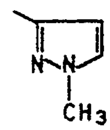 | 46 (Oxalat) |
| 26 | $CH_3$ | $CH_3$ | 2 | 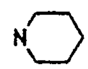 | 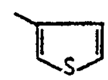 | 43 (Oxalat) |
| 27 | $CH_3$ | $CH_3$ | 2 | 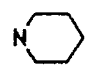 | 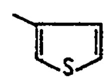 | 58 (Oxalat) |
| 28 | $CH_3$ | $C_2H_5$ | 2 | 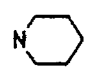 | 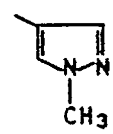 | Öl |
| 29 | $CH_3$ | $C_2H_5$ | 2 | 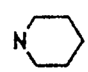 | 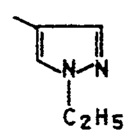 | 88 |
| 30 | $CH_3$ | $C_6H_5CH_2$ | 2 | 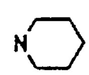 | 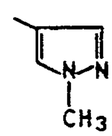 | 53 |

Tabelle 2 (Fortsetzung)

| Beisp. Nr. | $R^1$ | $R^2$ | n | $NR^3R^4$ | Het | Fp [°C] |
|---|---|---|---|---|---|---|
| 31 | $CH_3$ | $C_3H_7$ | 2 | 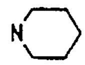 | 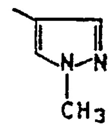 | Öl |
| 32 | $CH_3$ | $C_2H_5$ | 2 | 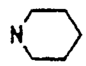 | 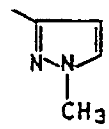 | 64 |
| 33 | $CH_3$ | $C_2H_5$ | 2 | 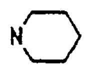 | 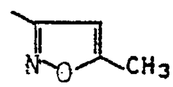 | Öl |
| 34 | $CH_3$ | $C_2H_5$ | 2 | 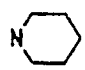 | 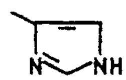 | Öl |
| 35 | $CH_3$ | $C_2H_5$ | 2 | 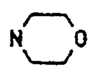 | 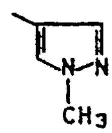 | Öl |
| 36 | $CH_3$ | $C_2H_5$ | 2 | 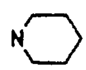 | 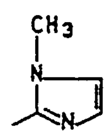 | Öl |

Tabelle 2 (Fortsetzung)

| Beisp. Nr. | $R^1$ | $R^2$ | n | $NR^3R^4$ | Het | Fp [°C] |
|---|---|---|---|---|---|---|
| 37 | $CH_3$ | $C_2H_5$ | 3 | 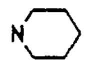 | 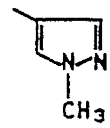 | Öl |
| 38 | $CH_3$ | $C_2H_5$ | 2 | 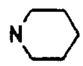 | 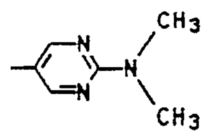 | Öl |
| 39 | $CH_3$ | $C_2H_5$ | 2 | 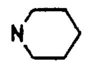 | 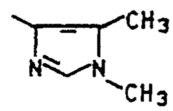 | 98 |
| 40 | $CH_3$ | $C_2H_5$ | 2 | 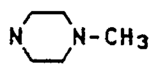 | 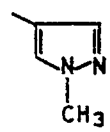 | Öl |
| 41 | $C_2H_5$ | $C_2H_5$ | 2 | 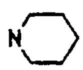 | 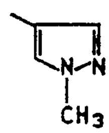 | Öl |
| 42 | $CH_3$ | $C_2H_5$ | 2 | 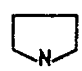 | 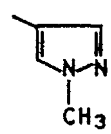 | Öl |
| 43 | $CH_3$ | $C_2H_5$ | 2 | $-N(CH_3)_2$ | 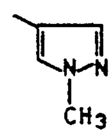 | Öl |
| 44 | $CH_3$ | $CH_3$ | 2 | 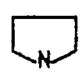 | 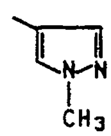 | Öl |

**Patentansprüche**

**Patentansprüche für folgende Vertragsstaaten : AT, BE, CH, DE, FR, GB, IT, LI, NL, SE**

**1.** Benzofuranderivate der allgemeinen Formel I

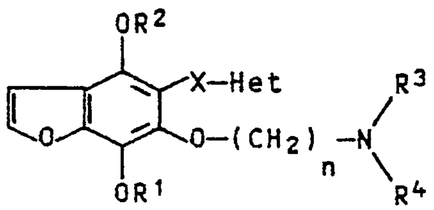

I,

wobei

| | |
|---|---|
| $R^1$ und $R^2$ | für Wasserstoff, Alkyl oder Phenylalkyl mit jeweils 1 bis 4 Kohlenstoffatomen im Alkylrest, |
| $R^3$ und $R^4$, | die gleich oder verschieden sind, für Wasserstoff oder $C_1$-$C_4$-Alkyl oder zusammen für eine 3- bis 5-gliedrige Kette, die durch ein Sauerstoffatom oder eine $NR^1$-Gruppe unterbrochen sein kann, |
| X | für $-CO-CH=CH-$, $-CO-CH_2-CH_2-$ oder $-CHOH-CH_2-CH_2-$, |
| n | für 2 oder 3 und |
| Het | für einen aromatischen 5- oder 6-gliedrigen Ring, der ein Heteroatom aus der Gruppe N, O und S und ggf. zusätzlich ein (weiteres) N-Atom enthält, jedoch nicht Pyridin, wobei der Ring substituiert sein kann durch $C_1$-$C_4$-Alkyl, Fluor, Chlor, Brom, $NH_2$ oder $NR^1R^2$, wobei $R^1$ und $R^2$ die obengenannte Bedeutung haben. |

2. Pharmazeutisches Mittel, neben üblichen galenischen Hilfsstoffen als Wirkstoff enthaltend eine Verbindung nach Anspruch 1.

3. Verwendung eines Benzofuranderivates nach Anspruch 1 zur Herstellung eines pharmazeutischen Mittels.

4. Verwendung eines Benzofuranderivat nach Anspruch 1 zur Herstellung eines antiulcerogenen Mittels.

**Patentanspruch für folgenden Vertragsstaat : ES**

1. Verfahren zur Herstellung eines antiulcerogenen Mittels durch übliche galenische Zubereitung eines entsprechenden Wirkstoffs, dadurch gekennzeichnet, daß man als Wirkstoff ein Benzofuranderivat der allgemeinen Formel I

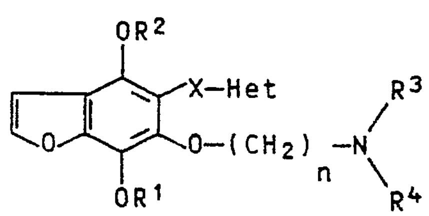

I,

wobei

| | |
|---|---|
| $R^1$ und $R^2$ | für Wasserstoff, Alkyl oder Phenylalkyl mit jeweils 1 bis 4 Kohlenstoffatomen im Alkylrest, |
| $R^3$ und $R^4$, | die gleich oder verschieden sind, für Wasserstoff oder $C_1$-$C_4$-Alkyl oder zusammen für eine 3- bis 5-gliedrige Kette, die durch ein Sauerstoffatom oder eine $NR^1$-Gruppe unterbrochen sein kann, |
| X | für $-CO-CH=CH-$, $-CO-CH_2-CH_2-$ oder $-CHOH-CH_2-CH_2-$, |
| n | für 2 oder 3 und |
| Het | für einen aromatischen 5- oder 6-gliedrigen Ring, der ein Heteroatom aus der Gruppe N, O und S und ggf. zusätzlich ein (weiteres) N-Atom enthält, jedoch nicht Pyridin, wobei der Ring substituiert sein kann durch $C_1$-$C_4$-Alkyl, Fluor, Chlor, Brom, |

$NH_2$ oder $NR^1R^2$, wobei $R^1$ und $R^2$ die obengenannte Bedeutung haben.

**Claims**

**Claims for the following Contracting States : AT, BE, CH, DE, FR, GB, IT, LI, NL, SE**

1. A benzofuran derivative of the formula I

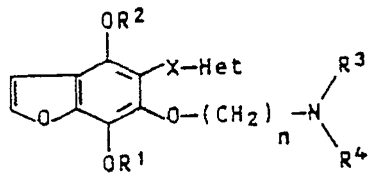

I

where $R^1$ and $R^2$ are each hydrogen, alkyl or phenylalkyl, where alkyl in each case is of 1 to 4 carbon atoms, $R^3$ and $R^4$ are identical or different and are each hydrogen or $C_1$-$C_4$-alkyl or together form a 3-membered to 5-membered chain which may be interrupted by an oxygen atom or an $NR^1$ group, X is -CO-CH=CH-, -CO-$CH_2$-$CH_2$- or -CHOH-$CH_2$-$CH_2$-, n is 2 or 3 and Het is an aromatic 5-membered or 6-membered ring which contains a hetero atom from the group consisting of N, O and S and may additionally contain a (further) N atom, but not pyridine, in which the ring may be substituted by $C_1$-$C_4$-alkyl, fluorine, chlorine, bromine, $NH_2$ or $NR^1R^2$, where $R^1$ and $R^2$ have the abovementioned meanings.

2. A pharmaceutical agent which contains a compound as claimed in claim 1 as an active compound in addition to conventional pharmaceutical auxiliaries.

3. Use of a benzofuran derivative as claimed in claim 1 for the preparation of a pharmaceutical agent.

4. Use of a benzofuran derivative as claimed in claim 1 for the preparation of an antiulcerogenic agent.

**Claim for the following Contracting State : ES**

1. A process for the preparation of an antiulcerogenic agent by conventional pharmaceutical formulation of an appropriate active compound, wherein the active compound used is a benzofuran derivative of the formula I

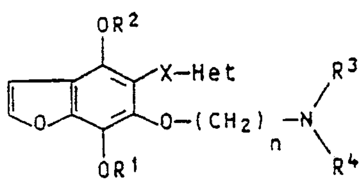

I

where $R^1$ and $R^2$ are each hydrogen, alkyl or phenylalkyl, where alkyl in each case is of 1 to 4 carbon atoms, $R^3$ and $R^4$ are identical or different and are each hydrogen or $C_1$-$C_4$-alkyl or together form a 3-membered to 5-membered chain which may be interrupted by an oxygen atom or an $NR^1$ group, X is -CO-CH=CH-, -CO-$CH_2$-$CH_2$- or -CHOH-$CH_2$-$CH_2$-, n is 2 or 3 and Het is an aromatic 5-membered or 6-membered ring which contains a hetero atom from the group consisting of N, O and S and may additionally contain a (further) N atom, but not pyridine, in which the ring may be substituted by $C_1$-$C_4$-alkyl, fluorine, chlorine, bromine, $NH_2$ or $NR^1R^2$, where $R^1$ and $R^2$ have the abovementioned meanings.

**Revendications**

**Revendications pour les Etats contractants suivants : AT, BE, CH, DE, FR, GB, IT, LI, NL, SE**

1. Dérivés de benzofuranne de formule générale I

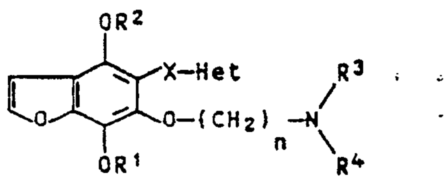

(I)

dans laquelle

$R^1$ et $R^2$ représentent hydrogène, alkyle ou phénylalkyle ayant chacun 1 à 4 atomes de carbone dans le reste alkyle,

$R^3$ et $R^4$, qui sont identiques ou différents, sont mis pour hydrogène ou alkyle en $C_1$-$C_4$ ou, ensemble, pour une chaîne à 3 à 5 chaînons, qui peut être interrompue par un atome d'oxygène ou un groupe $NR^1$,

X est mis pour $-CO-CH=CH-$, $-CO-CH_2-CH_2-$ ou $-CHOH-CH_2-CH_2-$,

n est mis pour 2 ou 3 et

Het pour un noyau aromatique à 5 ou six chaînons, qui contient un hétéroatome choisi dans le groupe composé de N, O et S, et éventuellement un (autre) atome N, mais non pas de la pyridine, le noyau pouvant être substitué par alkyle en $C_1$-$C_4$, fluor, chlore, brome, $NH_2$ ou $NR^1R^2$, $R^1$ et $R^2$ ayant les significations susmentionnées.

**2.** Produit pharmaceutique contenant, outre les produits auxiliaires galéniques usuels, un composé selon la revendication 1 comme principe actif.

**3.** Utilisation d'un dérivé de benzofuranne selon la revendication 1 pour la préparation d'un produit pharmaceutique.

**4.** Utilisation d'un dérivé de benzofuranne selon la revendication 1 pour la préparation d'un agent antiulcérogène.

**Revendication pour l'Etat contractant suivant : ES**

**1.** Procédé de préparation d'un agent antiulcérogène par préparation galénique usuelle d'un principe actif correspondant, caractérisé par le fait qu'on utilise comme principe actif un dérivé de benzofuranne de formule générale I

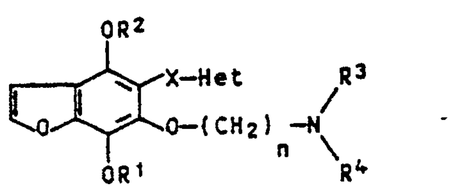

(I)

dans laquelle

$R^1$ et $R^2$ représentent hydrogène, alkyle ou phénylalkyle ayant chacun 1 à 4 atomes de carbone dans le reste alkyle,

$R^3$ et $R^4$, qui sont identiques ou différents, sont mis pour hydrogène ou alkyle en $C_1$-$C_4$ ou, ensemble, pour une chaîne à 3 à 5 chaînons, qui peut être interrompue par un atome d'oxygène ou un groupe $NR^1$,

x est mis pour $-CO-CH=CH-$, $-CO-CH_2-CH_2-$ ou $-CHOH-CH_2-CH_2-$,

n est mis pour 2 ou 3 et

Het pour un noyau aromatique à 5 ou six chaînons, qui contient un hétéroatome choisi dans le groupe composé de N, O et S, et éventuellement un (autre) atome N, mais non pas de la pyridine, le noyau pouvant être substitué par alkyle en $C_1$-$C_4$, fluor, chlore, brome, $NH_2$ ou $NR^1R^2$, $R^1$ et $R^2$ ayant les

significations susmentionnées.